# EUROPÄISCHE PATENTSCHRIFT

(11) **EP 2 679 186 B1**
(45) Veröffentlichungstag und Bekanntmachung des Hinweises auf die Patenterteilung: **09.09.2015**
(21) Anmeldenummer: 12173329.9
(22) Anmeldetag: 25.06.2012
(51) Int. Cl.: A61B 18/14, A61B 18/00

(54) **Instrument zur Gefäßfusion und -trennung**
Instrument for vessel fusion and separation
Instrument de fusion et de séparation des tissus

(43) Veröffentlichungstag der Anmeldung: 01.01.2014
(73) Patentinhaber: ERBE Elektromedizin GmbH, 72072 Tübingen (DE)
(72) Erfinder: Wandel, Waldemar, 72127 Kusterdingen (DE)
(74) Vertreter: Rüger, Barthelt & Abel

(56) Entgegenhaltungen:
- WO-A1-2006/021269
- WO-A1-2008/040483
- DE-T2- 60 226 015
- DE-U1-202007 009 165
- US-A1- 2002 099 371
- US-A1- 2003 199 869
- US-A1- 2011 054 468
- US-A1- 2012 095 460

## Beschreibung

Die Erfindung betrifft ein Instrument zum Abklemmen, Verschließen und Durchtrennen von Gewebe bzw. Gefäßen, insbesondere Blutgefäße am lebenden Körper eines menschlichen oder tierischen Patienten.

Prinzipiell sind Instrumente zum Verschluss und zur Trennung von Blutgefäßen bekannt. Ein Beispiel ist der DE 602 26 015 T2 zu entnehmen.

Solche Instrumente umfassen einen länglichen Schaft, an dessen distalen Ende ein Werkzeug mit zwei Branchen zum Abklemmen eines Blutgefäßes angeordnet ist. Außerdem ist dort ein längsverschiebbares Messer angeordnet, um ein gefasstes und koaguliertes Gefäß durchtrennen zu können. An dem proximalen Ende des Schafts ist ein Handgriff mit Betätigungsmitteln vorgesehen, über die die Branchen geschlossen und das Messer betätigt werden können. Die Branchen sind als Elektroden ausgebildet, um das zwischen ihnen geklemmte Gefäß erwärmen und die Gefäßwände durch Koagulation zur Fusion bringen zu können.

Aus der US 2012/0095460 A1 ist ein Instrument zur Gefäßfusion und Trennung bekannt, das einen länglichen Schaft aufweist, der sich von einem Gehäuse weg erstreckt. An dem Schaft sind zwei Branchen gehalten, von denen eine starr und die andere schwenkbar angeordnet sind. Beide Branchen sind mit Elektroden versehen, um ein Gefäß zwischen einander zu klemmen und zu koagulieren. Längs des Schafts ist ein Messer bewegbar, das in einem Schlitz der beweglichen Branche gelagert ist. Eine Schrägfläche der Branche dient dazu, das Messer in einen Schlitz der anderen Branche hinein zu fahren, um ein koaguliertes Gefäß zu durchtrennen. Auch US2011054468 und US2003199869 offenbaren ähnliche Instrumente.

Bei der Durchtrennung von Blutgefäßen muss sicher gestellt werden, dass die Enden des durchtrennten Gefäßes verlässlich geschlossen bleiben. Dabei müssen weitere Bedingungen erfüllt werden. Zum Beispiel soll die Schädigung umliegenden Gewebes vermieden werden. Der Gefäßverschluss soll weitgehend unabhängig von der Größe der Gefäße und der Dicke der Gefäßwände erreichbar sein. Dabei muss im Weiteren auf die beengten Platzverhältnisse Rücksicht genommen werden, die spätestens dann zu beachten sind, wenn das Instrument endoskopisch oder laproskopisch eingesetzt werden soll.

Davon ausgehend ist es Aufgabe der Erfindung, ein Instrument zur Gefäßfusion und -trennung zu schaffen, mit dem sich Gefäße in hoher Qualität verschließen und trennen lassen.

Diese Aufgabe wird mit dem Instrument nach Anspruch 1 gelöst:
Das erfindungsgemäße Instrument dient dem Verschließen und Durchtrennen von Blutgefäßen. Es weist zwei Branchen auf, von denen wenigstens eine in Bezug auf die andere beweglich, vorzugsweise schwenkbar, gelagert ist. Es können beide Branchen bezüglich des Schafts des Instruments beweglich gelagert sein. Es ist auch möglich, dass eine der Branchen starr mit dem Schaft verbunden ist während die andere beweglich gelagert ist. Letzteres wird gegenwärtig bevorzugt. Die Lagerung der Brachen kann so ausgeführt sein, dass nicht uniformes Gewebe leicht gefasst werden kann.

Die erste Branche weist vorzugsweise einen elektrisch isolierenden bspw. aus Keramik oder auch einen geeigneten Kunststoff bestehenden Grundkörper mit einer rinnenartigen Ausnehmung für eine Elektrode und einem Führungsschlitz für ein Messer auf. Die rinnenartige Ausnehmung kann der Form der Elektrode angepasst sein. Die Elektrode weist vorzugsweise eine konkave Ausnehmung auf. Sie ist bspw. als Zylinderschale ausgebildet. Sie kann jedoch auch einen U-Querschnitt mit geraden Schenkeln aufweisen. Weiter alternativ kann sie als ebene Elektrode ausgebildet sein. Vorzugsweise wird sie durch ein dünnwandiges Blechteil gebildet, das durch die Abstützung in dem Grundkörper die nötige mechanische Stabilität erhält.

Die Elektrode ist über ein elektrisches Anschlussmittel mit einer sich durch den Schaft erstreckenden Leitung verbunden. Das Anschlussmittel ist ein vorzugsweise isolierter elektrischer Leiter. Er führt durch geeignete Kanäle oder Durchgänge des Grundkörpers und kann z.B., insbesondere in der Nähe eines Gelenks, bei dem die zweite Branche beweglich an der ersten Branche gelagert ist, an der Außenseite des Grundkörpers verlaufen. Der Grundkörper kann dazu mit einer längs verlaufenden Ausnehmung versehen sein, in der die Leitung angeordnet ist. Dadurch kann sichergestellt werden, dass der Kopf des Instruments einen maximalen Außenquerschnitt nicht übersteigt und das Instrument z.B. zum laproskopischen Einsatz durch einen Trokar geschoben werden kann.

Vorzugsweise wird als elektrischer Leiter ein physiologisch verträgliches Material wie Silber, Titan oder dergleichen genutzt. Der Leiter kann als massiver Runddraht, Hohldraht, als Flachdraht, als Litze, als Band oder dergleichen ausgeführt sein.

Das Messer ist bezüglich seiner Vorschubrichtung präzise geführt. Vorzugsweise ist das Messer dabei mittig zu der Elektrode und auch mittig zu der zweiten Branche geführt, in deren Längsschlitz sie einfahren kann. Die Führung des Messers kann beim Vorschieben zumindest teilweise von dem Längsschlitz der zweiten Branche geleistet werden.

Durch die präzise Führung des Messers kann sichergestellt werden, dass der Schnitt beim Durchtrennen eines Gefäßes mittig in dem zuvor fusionierten Bereich des Gefäßes gesetzt wird. Damit haben beide Enden des schlussendlich durchtrennten Gefäßes gleich lange fusionierte Abschnitte. Die Gefahr, dass es wegen eines nicht präzise mittig gesetzten Schnittes zu unkontrollierten Blutungen kommt, ist minimiert.

Erfindungsgemäß weist das Instrument einen Vertikalstellmechanismus auf, der dem Messer zugeordnet ist. Er bewirkt eine Bewegung des Messers in Abhängigkeit von seiner Längsbewegung senkrecht zu dieser. Diese Bewegung ist senkrecht zu der Elektrode gerichtet und wird deshalb unabhängig von der absoluten räumlichen Ausrichtung als "Vertikalbewegung" bezeichnet. Durch die der Längsbewegung überlagerte Vertikalbewegung des Messers wird seine Spitze und die sich daran anschließende Gleitkante geschont. Die Spitze wird erst dann auf die vorzugsweise aus Metall bestehende Elektrode abgesenkt, wenn die Spitze (und vorzugsweise auch die sich anschließende Schneidkante) bezüglich der Längsbewegung vor der Elektrode liegende, z.B. aus Keramik bestehende Abschnitte, Stufen, Fugen oder dergleichen überquert hat. Die Spitze und die Schneidkante des Messers werden so berührungslos an ein Ende der Elektrode geführt und dann auf die Elektrode aufgesetzt. Ein vorzeitiges Abstumpfen der Schneide und/oder der Spitze wird dadurch vermieden. Dies stellt einen sauberen Schnitt des zu trennenden Gewebes bzw. Gefäßes sicher.

Vorzugsweise weist der Vertikalstellmechanismus eine z.B. an dem Grundkörper angeordnete Kurvenfläche auf, der eine Kurvenfolgerfläche zugeordnet ist. Diese ist mit dem Messer oder einem Messerträger verbunden. Die Orientierung der Flächen ist schräg zu der Bewegungsrichtung. Dadurch leiten die beiden Flächen die gewünschte Vertikalstellbewegung aus der Längsbewegung des Messers ab. Vorzugsweise ist der Abstand der Kurvenfolgerfläche von dem Gleitkantenende wenigstens so groß ist, wie der Abstand der Kurvenfläche von der Fuge oder Stufe. Dadurch wird das Messer sicher über die Stufe geführt.

Vorzugsweise umfasst das Messer eine Führungsleiste und eine Klinge, die fest miteinander verbunden sind. Die Führungsleiste ist vorzugsweise ein längliches sich in Bewegungsrichtung des Messers erstreckendes Element zum Beispiel in Form einer Kunststoffschiene. Sie kann mit einer Klinge aus Metall verbunden sein. Die Klinge kann jedoch auch aus Keramik, Diamant, Kunststoff oder sonstigem Material bestehen. Die Führungsleiste kann alternativ aus einem Metall bestehen. Es ist auch möglich, die Führungsleiste durch eine an der Klinge ausgebildete Verdickung zu bilden, so dass Führungsleiste und Klinge nahtlos einteilig miteinander aufgebaut sind. Der zweiteilige Aufbau wird aber bevorzugt.

Das seitliche Spiel des Messers in dem Führungsschlitz und/oder in dem Längsschlitz ist vorzugsweise geringer als das Fünffache der Dicke D der Klinge. Vorzugsweise ist das seitliche Spiel des Messers höchstens etwa so groß wie die Dicke der Klinge. Dadurch wird eine präzise mittige Führung des Messers und somit eine mittige Schnittführung im fusionierten Gewebebereich erzielt. Die seitliche Führung des Messers kann durch den Führungsschlitz erbracht werden. Die seitliche Führung des Messers kann alternativ durch den Längsschlitz erbracht werden. Vorzugsweise wird die seitliche Führung sowohl durch den Führungsschlitz als auch durch den Längsschlitz erbracht.

Unter seitlichem Spiel des Messers wird der Abstand der Flanken der Führungsleiste von der Flanke des Führungsschlitzes oder Längsschlitzes bei mittiger Messerlage verstanden. Die seitliche Beweglichkeit des Messers entspricht somit dem doppelten Spiel.

Vorzugsweise weist das Messer eine gerade, der Elektrode zugewandte und an dieser anliegende längs verlaufende Gleitkante auf. Die Gleitkante kann relativ kurz ausgebildet sein und schließt sich vorzugsweise unmittelbar an die Schneidkante des Messers an. Somit wird sichergestellt, dass das zwischen den Branchen gefasste Gefäß auf ganzem Querschnitt durchtrennt wird und kein Geweberest undurchtrennt bleibt.

Die Schneidkante ist an dem Messer vorzugsweise stirnseitig angeordnet. Sie steht vorzugsweise unter einem Winkel zu der Bewegungsrichtung des Messers. Dieser Winkel kann ein spitzer Winkel sein. Vorzugsweise ist die Schneidkante gerade ausgebildet, wobei sie auf die Führungsleiste hin läuft. Vorzugsweise überragt die Führungsleiste die Schneidkante, um zu verhindern, dass Teile des zu durchtrennenden Gefäßes über das Messer gedrängt und somit vom Schnitt nicht erfasst werden.

Die zweite Branche weist, zumindest an ihrer der Elektrode zugewandten Seite, eine elektrisch leitende Fläche auf. Ist die zweite Branche aus einem nicht metallischen Material kann diese Fläche durch ein dünnes Elektrodenblech gebildet sein. Vorzugsweise aber ist die zweite Branche massiv aus Metall ausgebildet. Unabhängig davon weist die zweite Branche an ihrer der Elektrode zugewandten Seite eine der Form der Elektrode folgende Form auf. Mit anderen Worten, zwischen der Elektrode und der zweiten Branche wird ein Abstandsspalt von im Wesentlichen konstanter oder zur Mitte hin abnehmender Spaltweite gebildet. Unabhängig davon kann die Oberfläche der zweiten Branche profiliert, bspw. gerippt, genoppt oder anderweitig geformt sein. Auch die Elektrode kann mit einer Oberflächenprofilierung versehen sein. Auch wenn die Profilierungen der Elektrode und der zweiten Branche gegebenenfalls unterschiedlich sind, haben sie vorzugsweise zueinander passende einander folgende Grundformen zur Festlegung eines Abstandsspalts mit vorzugsweise zur Mitte hin abnehmender Spaltweite.

Die Haltemittel zur Fixierung der Elektrode an der ersten Branche sind vorzugsweise an den oberen, einander gegenüber liegenden Rändern der Ausnehmung ausgebildet. Vorzugsweise sind diese Haltemittel durch elektrisch isolierende Vorsprünge gebildet, die bspw. aus dem Material des Grundkörpers bestehen. Vorzugsweise ragen sie in Richtung auf die zweite Branche zu und überdecken somit den oberen Rand der Elektrode. Vorzugsweise legen die Vorsprünge mit der zweiten Branche bei geschlossenem Instrument einen Spalt mit einem Abstand B fest, der höchstens so groß ist, wie die Weite A des Abstandsspalts in Nachbarschaft zu den Haltemitteln.

Zwischen der zweiten Branche und solchen Vorsprüngen kann Gewebe des abzuklemmenden Gefäßes gefasst werden, das wegen der elektrisch isolierenden Eigenschaften der Vorsprünge hier nicht koaguliert wird. Das Fassen des Gewebes ist auch dann sichergestellt, wenn die Brachen aufgrund von Gewebeeigenschaften nicht symmetrisch schließen. Wird beispielesweise ein Gefäß gefasst, dessen Querschnitt sich über die Länge verändert, kann dies dazu führen, dass die seitlichen Abstände B unterschiedliche Werte aufweisen können d.h. nicht gleich groß sind. Trotzdem wird das Gefäß sicher gehalten. Ein Verrutschen des Gefäßes ist auch dann sicher ausgeschlossen, wenn das zwischen der Branche und der Elektrode gefasste Gewebe durch Wärmeeinwirkung und Koagulation schrumpft und dort die Klemmung abnimmt. Ein seitliches Verrutschen des Gefäßes und infolgedessen ein späteres Durchtrennen des Gefäßes an ungeeigneter Stelle bei nachfolgender Aktivierung des Messers wird auf diese Weise sicher ausgeschlossen.

Die z.B. durch die Vorsprünge gebildeten Haltemittel haben eine Doppelfunktion: Sie halten die Elektrode und sie Verbessern das Halten des Gefäßes.

Zwischen der ersten und der zweiten Branche ist vorzugsweise ein Abstandhaltemittel vorgesehen. Dieses Abstandshaltemittel verhindert, dass die zweite Branche die Elektrode berührt und es somit zum Kurzschluss kommt. Außerdem verhindert das Abstandhaltemittel, dass unbeabsichtigt ein zu großer Druck auf das Gefäß ausgeübt und dieses somit abgequetscht wird. Vorzugsweise ist das Abstandshaltemittel durch einen metallischen Vorsprung gebildet, dem ein nicht metallischer Anschlag auf der Gegenseite zugeordnet ist. Der metallische Vorsprung ist vorzugsweise elektrisch beaufschlagt, d.h. mit der Elektrode oder mit der zweiten Branche verbunden. Dadurch werden Fehler vermieden, die auftreten könnten, wenn biologisches Gewebe zwischen den Vorsprung und die ihm zugeordnete Anlagefläche gelangt. Durch die elektrische Bestromung kann, insbesondere wenn der Vorsprung in der Nähe der Gegenpolelektrode angeordnet ist, ein gewisser Stromfluss in das gefasste biologische Gewebe eingeleitet werden, so dass es schrumpft und die Einstellung des gewünschten Minimalabstands zwischen der zweiten Branche und der Elektrode nicht mehr behindert. Bei einem alternativen Ausführungsbeispiel ist es möglich, dass das Abstandshaltemittel aus einem elektrisch nicht leitenden Material gebildet ist. Dadurch kann die Kurzschlussgefahr zwischen den Branchen reduziert werden. Zusätzlich ermöglicht dies, den Koagulationsbereich zu erweitern.

Weitere Einzelheiten vorteilhafter Ausführungsformen der Erfindung ergeben sich aus der nachfolgenden Beschreibung, Unteransprüchen oder der Zeichnung. Es zeigen:
Figur 1 ein erfindungsgemäßes Instrument in schematisierter Darstellung.
Figur 2 die Branchen und das Messer des Instruments, in schematisierter Seitenansicht.
Figur 3 die Branchen und das Messer gemäß Figur 2, in teilweise aufgeschnittener Prinzipansicht.
Figur 4 die Branchen und das Messer, in Vertikalschnittdarstellung mit Blick in Richtung der Längsachse, in vereinfachter, schematisierter Darstellung bei geschlossenem Werkzeug,
Figur 5 den Kopf des erfindungsgemäßen Instruments, in perspektivischer Ansicht,
Figur 6 den Grundkörper und das Messer, in Vertikalschnittdarstellung mit Blick in Richtung der Längsachse, in vereinfachter, schematisierter Darstellung,
Fig. 7 - 9 den Kopf des erfindungsgemäßen Instruments nach Figur 5 in längs geschnittener Darstellung in drei verschiedenen Positionen des Messers,
Figur 10 einen Vertikalstellmechanismus für das Messer, in schematischer Darstellung, und
Figur 11 das Messer in Ruheposition und die zugehörige Elektrode, in seitlicher schematischer Ansicht.

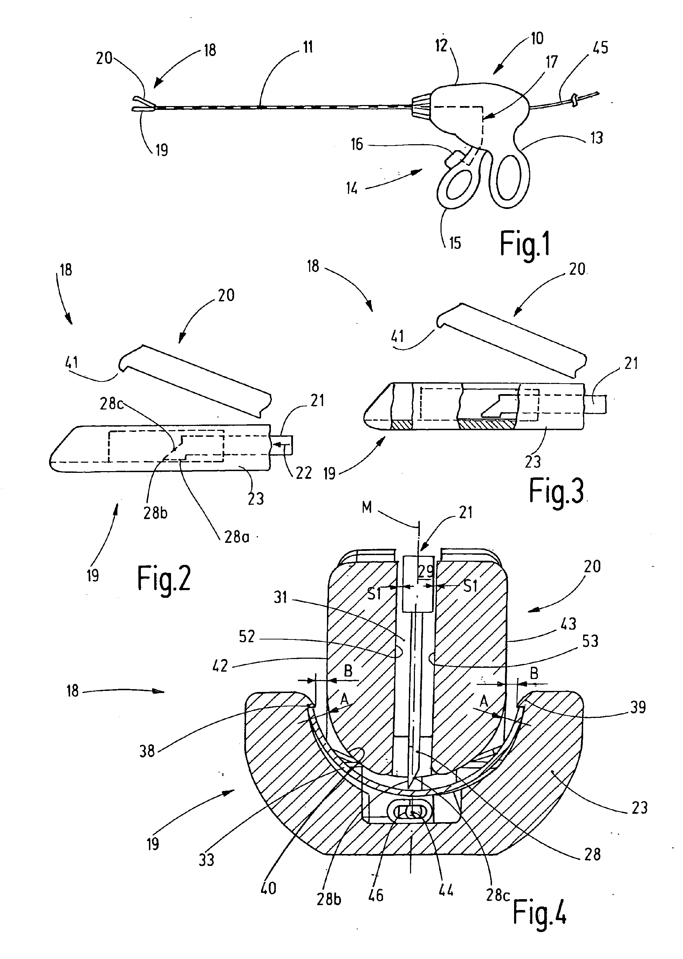

In Figur 1 ist ein Instrument 10 veranschaulicht, das zum Abklemmen, Fusionieren und Durchtrennen eines Gefäßes z.B. eine Blutgefäßes beim Operieren an einem menschlichen oder tierischen Körper dienen kann. Das Instrument 10 dient insbesondere der endoskopischen Chirurgie. Es umfasst einen schlanken Schaft 11, der an seinem proximalen Ende an einem Gehäuse 12 gehalten ist. Dieses weist Handhabungsmittel beispielsweise einen Handgriff 13 sowie Betätigungselemente 14 auf. Diese können z.B. aus einem schwenkbaren Griffteil 15 und/oder einem Betätigungsknopf- oder -hebel 16 sowie gegebenenfalls weiteren Elementen, z.B. elektrischen Schaltern bestehen. Die Betätigungselemente 14 sind über in Figur 1 lediglich schematisch gestrichelt angedeutete Kraftübertragungsmittel 17 mit einem an dem distalen Ende des Schafts 11 angeordneten Anwendungsteil 18 verbunden. Dieses Anwendungsteil oder Kopf 18 des chirurgischen Instruments umfasst mindestens die in den Figuren 2 und 3 veranschaulichten Elemente, nämlich eine erste Branche 19, eine zweite Branche 20 und ein Messer 21. Die Branchen 19, 20 dienen zum Abklemmen eines Gefäßes, indem sie aufeinander zu bewegt werden und das Gefäß zwischen einander festklemmen. Das Messer 21 dient dazu, das Gefäß zu durchtrennen.

Im vorliegenden Ausführungsbeispiel ist die Branche 19 starr mit dem Schaft 11 verbunden, während die Branche 20 durch Betätigung des Griffteils 15 auf die Branche 19 hin oder von dieser weg zu schwenken ist. Hingegen wird das Messer 21 durch Betätigung eines Betätigungselementes, beispielsweise des Griffteils 15, in Längsrichtung des Schafts 11, wie in Figur 2 durch einen Pfeil 22 angedeutet, vorwärts geschoben.

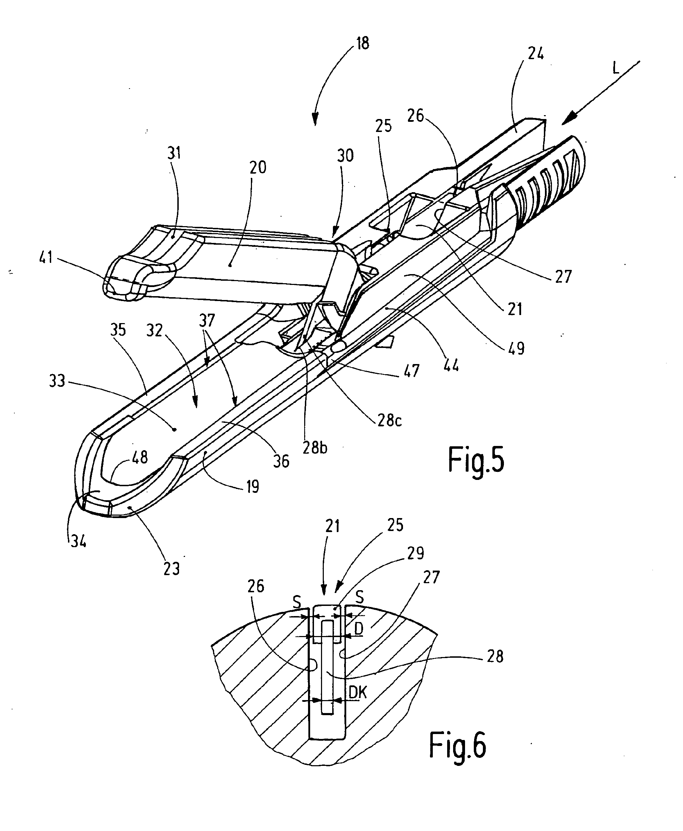

Die Branche 19 ist mehrteilig aufgebaut. Sie umfasst einen z.B. aus Kunststoff oder Keramik bestehenden Grundkörper 23, wie er insbesondere aus Figur 4 ersichtlich ist. Der Grundkörper 23 ist an seinem schaftseitigen Ende mit dem Schaft 11 starr verbunden, wozu er einen aus Figur 5 ersichtlichen Ansatz 24 aufweist. Von diesem ausgehend erstreckt sich der längliche Grundkörper 23 weg, wobei er zunächst einen Führungsschlitz 25 für das Messer 21 aufweist. Der sich in Längsrichtung L durch den länglichen Grundkörper 23 erstreckende Führungsschlitz 25 weist zwei Schlitzflanken 26, 27 auf, die einander parallel im Abstand gegenüber liegen und zwischen einander das Messer 21 führen. Dies ist insbesondere in Figur 6 veranschaulicht. Das Messer 21 hat dabei in Mittellage zu jeder Schlitzflanke 26, 27 einen als Spiel S bezeichneten Abstand, der jeweils vorzugsweise höchsten so groß ist, wie die Dicke D des Messers 21.

Letzteres besteht aus einer Klinge 28, die an ihrer oberen Kante mit einer Führungsleiste 29 versehen ist. D ist dabei als die Dicke der Führungsleiste 29 definiert (Figur 6). Als Dicke DK ist die Dicke der Klinge 28 definiert. Vorzugsweise ist das Spiel S höchsten so groß wie das Fünffache der Dicke DK. Im besseren Fall ist die Dicke DK höchstens so groß wie das Doppelte des Spiels S. Mit anderen Worten, das Messer 21 kann sich um maximal ein Maß von zweimal S zwischen den Flanken 26, 27 hin und her bewegen. Im Idealfall ist die Dicke DK höchstens so groß wie das Spiel S.

Das Messer 21 weist vorzugsweise eine z.B. an der Klinge 28 angeordnete Gleitkante 28a auf, die an einer Elektrode 33 der Branche 19 gleiten kann und das Messer 21 in der Höhe führt (Figur 2). Dadurch kann die sich unten an die Schneidkante 28c anschließende Spitze 28b der Klinge 28 (Figur 4 und 5)an der Elektrode 33 oder mit ganz geringem Abstand zu dieser laufen, ohne sich zu verschleißen.

In gerader Verlängerung an den Führungsschlitz 25 schließt sich ein Lager- oder Scharnierbereich 30 an, in dem die zweite Branche 20 schwenkbar an dem Grundkörper 23 gelagert ist. Die Branche 20 ist mit dem in Figur 5 nicht weiter veranschaulichten Kraftübertragungsmittel 17 verbunden, um durch dieses um eine quer zu dem Grundkörper 23 stehender Achse geschwenkt zu werden.

Der Führungsschlitz 25 setzt sich als Längsschlitz 31 durch die Branche 20 fort. Der Längsschlitz 31 ist, zumindest nach unten, d.h. nach unten zu dem Grundkörper 23 hin offen. Vorzugsweise stimmt seine Breite wenigstens näherungsweise mit der Breite des Führungsschlitzes 25 überein. Die Schlitzflanken 52, 53 des Längsschlitzes 31 sind ebene, parallel zueinander orientierte Flächen. Mit dem Messer 21 definieren sie die Spielmaße S1 für die das oben zu dem Spiel S gesagte entsprechend gilt.

Der Grundkörper 23 weist unterhalb der Branche 20 eine rinnen- oder muldenförmige Ausnehmung 32 auf, in der die Elektrode 33 angeordnet ist. Die Elektrode 33 wird im vorliegenden Ausführungsbeispiel durch ein dünnes zylinderschalenförmiges Blech gebildet. Sie weist an der der Branche 19 zugewandten Seite eine gekrümmte, vorzugsweise konkave, vorzugsweise geschlossene, glatte Fläche auf. Die Elektrode 33 erstreckt sich dabei über nahezu die gesamte Länge der Ausnehmung 32, wobei sie jedoch an dem vorderen Ende des Grundkörpers 23 einen Bereich 34 frei lässt. An den oberen Rändern 35, 36 des Grundkörpers 23 sind Haltemittel 37 für die Elektrode 33 angeordnet. Diese sind, wie Figur 4 zeigt, durch Rastvorsprünge 38, 39 gebildet, die aus elektrisch isolierendem Material bestehen und vorzugsweise einstückiger Bestandteil des Grundkörpers 23 sind. Die Rastvorsprünge 38, 39 bilden Haltenasen, die den oberen Rand der Elektrode 33 übergreifen. Vorzugsweise übersteigt ihre Länge etwas die Dicke der Elektrode 33, so dass sie deren freiliegende Elektrodenfläche etwas überragen.

Die obere Branche 20 weist an ihrer der Elektrode 33 zugewandten Unterseite ein der Form der Elektrode 33 folgende Elektrodenfläche 40 auf. Ist die Elektrode 33 bspw. zylinderschalenförmig ausgebildet, folgt die Elektrodenfläche 40 ebenfalls einem Kreisbogen, wobei dieser einen etwas geringeren Radius aufweist. Unabhängig von dieser Grundform können sowohl die Elektrode 33 als auch die Elektrodenfläche 40 lokale Formabweichungen in Form von Ausnehmungen, Vorsprüngen, Noppen, Rippen oder dergleichen aufweisen.

An dem distalen Ende der zweiten Branche 20 ist ein sich zu dem Grundkörper 23 hin erstreckender nasenartiger Vorsprung 41 vorgesehen, der sich bei geschlossenem Instrument an dem endständigen Bereich 34 des Grundkörpers 23 abstützt. In diesem Zustand ist zwischen der Elektrodenfläche 40 und der Innenseite der Elektrode 33 ein Abstandsspalt mit der Weite A festgelegt. Vorzugsweise ist diese Weite A, zumindest geringfügig, größer als der Abstand B zwischen dem Vorsprung 38 bzw. 39 und der Elektrodenfläche 40 oder sich daran anschließenden Flanke 42, 43 der Branche 20.

Die Elektrode 33 ist, wie aus den Figuren 4, 5 ersichtlich, über einen elektrischen Leiter 44 an eine elektrische Quelle angeschlossen. Der Leiter 44 führt dabei durch den Schaft 11 zunächst in das Gehäuse 12 dort zu einem nicht weiter veranschaulichten Schalter und gegebenenfalls über eine Zuleitung 45 zu einem versorgendem Gerät.

Ebenso führt von der Branche 20 ein nicht weiter veranschaulichter Leiter zu dem Gerät. Der Leiter 44 kann bspw. ein mit einer Isolierung 46 versehener Runddraht oder dergleichen sein. Er ist etwa zentral an einer zwischen der Elektrode 33 und dem Grundkörper 23 liegenden Stelle an die Elektrode 33 angeschlossen. Insbesondere im Scharnierbereich 30 ist der Leiter 44 durch eine seitliche Öffnung 47 des Grundkörpers 23 an dessen Außenseite geführt und verläuft dort in einer Längsausnehmung 49 bis zu dem Ansatz 24. Dort tritt der Leiter 44 in den Schaft 11 ein. Der Leiter 44 kann insbesondere ein isolierter Silberdraht sein.

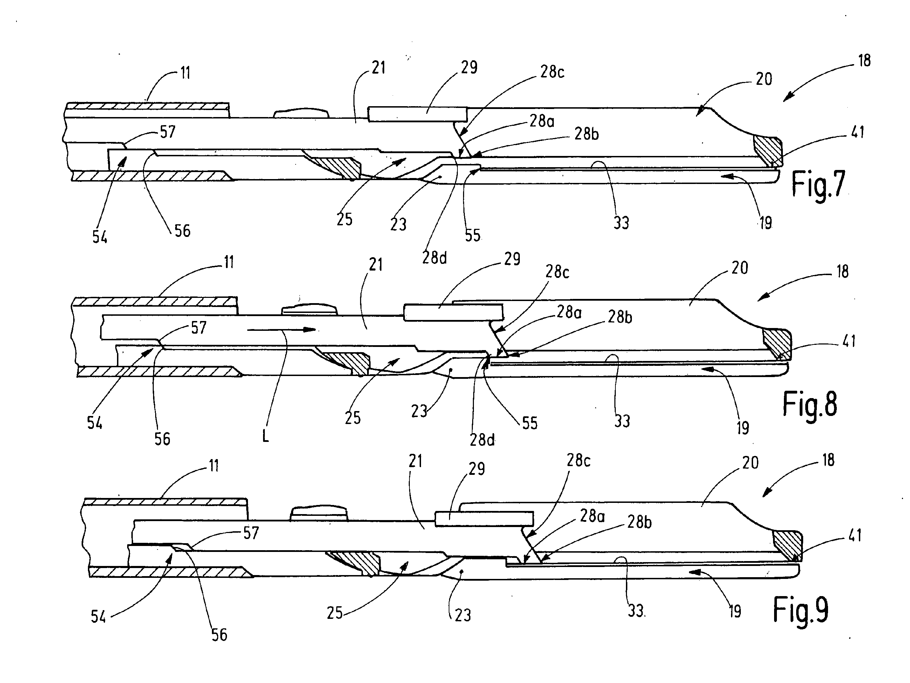

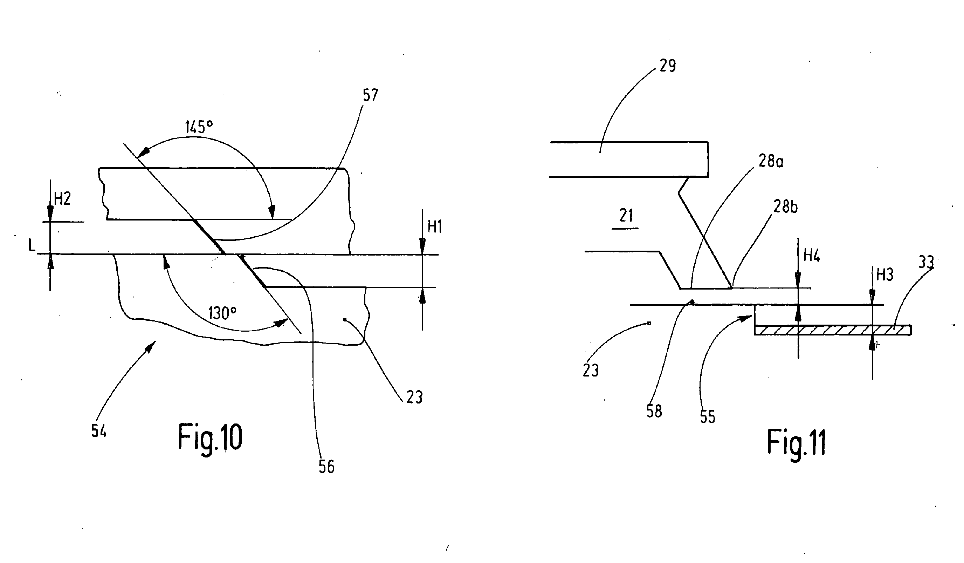

Dem Messer 21 ist ein Vertikalstellmechanismus 54 zugeordnet, der dazu dient, dem Messer 21 eine senkrecht zu der Elektrode 33 hin bzw. von der Elektrode 33 weg gerichtete Bewegung zu erteilen. Diese Bewegung ist quer (in den Figuren 7 bis 11 senkrecht) zu der entlang der Längsrichtung L stattfindenden Längsbewegung des Messers 21 gerichtet. Der Vertikalstellmechanismus 54 dient dazu, aus der Längsbewegung des Messers 21 eine vertikale Stellbewegung abzuleiten. Der Vertikalstellmechanismus 54 hält das Messer 21, wenn es sich in der Rückzugsposition gemäß Figur 7 befindet, in einer angehobenen Position, in der seine Spitze 28b und seine Gleitkante 28a, wie es Figur 11 zeigt, so weit von der Elektrode 3 abgehoben sind, dass selbst eine sich an ein Ende der Elektrode 33 anschließende Stufe 55 nicht berührt wird. Der Vertikalstellmechanismus 54 ist in Figur 10 gesondert veranschaulicht. Zu ihm gehören eine Kurvenfläche 56, die z.B. an dem Grundkörper 23, dem Ansatz 24 oder einem fest mit dem Grundkörper 23 verbundenen Teil ausgebildet sein kann, sowie eine Kurvenfolgerfläche 57, die an dem Messer 21 oder einem mit diesem fest verbundenen Teil ausgebildet ist. Die Kurvenfläche 56 und die Kurvenfolgerfläche 57 können als ebene Flächen oder auch anderweitig, beispielsweise als in Seitenansicht S-förmige oder bogenförmig gewölbte Flächen ausgebildet sein. Sie sind unabhängig von ihrer konkreten Form schräg zu der Längsrichtung L orientiert. Wie Figur 10 andeutet, eignet sich beispielsweise ein Winkel von 130° zu der Längsrichtung L. Die Kurvenfolgerfläche 57 kann in einem gleichen oder auch einem abweichenden Winkel zur Längsrichtung L geneigt sein. Es eignet sich beispielsweise ein Winkel von 145°. Andere Winkel sind möglich. Die Kurvenfläche 56 ist quer zu der Längsrichtung L orientiert und weist vorzugsweise eine Höhe H1 von zum Beispiel 0,4 mm auf. Die Kurvenfolgerfläche 57 hat vorzugsweise, gemessen quer zur Längsrichtung L, eine etwas größere Höhe H2 von beispielsweise 0,5 mm. Die Höhe H1 ist vorzugsweise deutlich größer als die Höhe H3 der Stufe 55, wie sie aus Figur 11 zu entnehmen ist. Die Höhe H3 kann beispielsweise 0,2 mm betragen. Damit wird ein Mindestabstand H4 der Spitze 28b und der Gleitkante 28a von der sich an die Stufe 55 anschließenden Fläche 58 von beispielsweise 0,1 mm bis 0,2 mm eingehalten.

Das insoweit beschriebene Instrument 10 arbeitet wie folgt:
Zum Schließen und Trennen eines Gefäßes wird dieses zwischen den Branchen 19, 20 gefasst. Durch Betätigung des Griffteils 15 werden die Branchen 19, 20 aufeinander zu bewegt, so dass das Gefäß abgeklemmt wird und die Gefäßwände aneinander gedrückt werden. Die Gefäßwände werden dabei insbesondere zwischen der Elektrodenfläche 40 und der Elektrode 33 zusammengedrückt. Sie werden zusätzlich zwischen den Vorsprüngen 38, 39 und der Elektrodenfläche 40 oder den Flanken 42, 43 gefasst. Im Idealfall sitzt der Vorsprung 41 auf dem als Widerlager dienenden Bereich 34 des Grundkörpers 23 und verhindert somit ein Abquetschen des Gefäßes.

Durch Anlegen eines Stroms oder Spannung zwischen der Branche 20 und der Elektrode 33, vorzugsweise einer HF-Spannung wird ein Koagulationsprozess eingeleitet, in dessen Verlauf Zellen der Gefäßwände geöffnet werden sowie Eiweiß freigesetzt und denaturiert wird. Es kommt zum Verkleben der zwischen der Branche 20 und der Elektrode 33 gefassten aneinander gedrückten Gefäßwände. Diese können dabei an Volumen verlieren. Unabhängig vom Schwinden des Volumens der Gefäßwände bleiben jedoch die zwischen den Vorsprüngen 38, 39 und der Branche 20 gefassten Partien der Gefäße, zumindest weitgehend, in natürlichem Zustand und unterliegen somit kaum einer Schwindung. Sie bleiben sicher gefasst, wodurch ein seitliches Verrutschen des Gefäßes verhindert wird. Dies hat insbesondere beim Schließen relativ kleiner Gefäße Bedeutung.

Ist hingegen ein Geweberest zwischen die Nase 41 und den als Widerlager dienenden Bereich 34 geraten, so dass die Spaltweite A etwas größer als eigentlich vorgesehen ist, wirkt wiederum der enge Spalt B als Sicherung gegen seitliches Verrutschen des Gefäßes. Außerdem kann der Abstand zwischen der Nase 41 und dem vorderen Ende 48 der Elektrode 33 so gering sein, dass hier vorhandenes Gewebe koaguliert wird und somit schwindet. Auch dies kommt wiederum dem sicheren Fassen des Gewebes zwischen den Branchen 19, 20 zugute.

Ist der Koagulationsprozess ausreichend weit fortgeschritten, kann der Chirurg z.B. durch Betätigung des Griffteils 15das Messer 21 aktivieren. Es wird nun ausgehend von der Position nach Figur 7 in dem Führungsschlitz 25 und dem Längsschlitz 31 mittig geführt in Längsrichtung L zu dem freien Ende der Branchen 19, 20 hin geschoben. Das Messer 21 läuft dabei mehr oder weniger genau innerhalb einer Mittelebene M, die in Figur 4 strichpunktiert angedeutet ist. Damit wird sichergestellt, dass zu beiden Seiten des Messers 21 gleich lange Bereich geschlossener Gefäßenden entstehen. Die Gefahr eines außermittigen Schnitts und somit die Gefahr unkontrollierter Blutungen ist dadurch minimiert.

In Ruheposition steht das Messer 21 zunächst gemäß Figur 7 mit seiner Spitze 28a aus Sicht der Elektrode 33 hinter der Stufe 55, an welcher der aus Keramik oder einem anderen Material bestehende Grundkörper 23 bei einer Fuge an die Elektrode 33 anschließt. Der Abstand des hinteren (in Figur 7 linksseitigen) Endes 28d der Gleitkante 28a von der Stufe 55 ist dabei etwas geringer als der Abstand zwischen der Kurvenfläche 56 und der Kurvenfolgerfläche 57. Die Position der Gleitkante 28a gemäß Figur 7 ist in Figur 11 gesondert veranschaulicht. Mit zunehmendem Vorschub des Messers 21 gelangt dieses in die Position nach Figur 8. Kurz nachdem das spitzenferne Ende 28d die Stufe 55 passiert hat, gelangt die Kurvenfolgerfläche 57 in Anlage mit der Kurvenfläche 56. Es begibt nun eine Absenk- oder Zustellbewegung im Rahmen derer, das Messer 21 quer zu der Längsrichtung L an die Elektrode 33 herangeführt wird. Dazu steht das Messer 21 durch die Elastizität seiner nicht weiter veranschaulichten Halterung oder der Kraftübertragungsmittel 17 in einer gewissen Vorspannung auf die Elektrode 33 hin. Die Gleitkante 28a setzt nun auf der Elektrode 33 auf, wie es Figur 9 zeigt. Beim weiteren Vorschub kann die Schneidkante 28c das gefasste und koagulierte Gefäß trennen.

Das Abheben der Gleitkante 28a beim Rückzug des Messers 21 erfolgt entsprechend umgekehrt. Jedenfalls wird die Gleitkante 28a ebenso wie die Spitze 28b vor der Berührung mit tendenziell abrasiveren Materialien, beispielsweise Keramikteilen, bewahrt, so dass die Schärfe insbesondere der Spitze 28b erhalten bleibt. Dies stellt die Erzielung einer hohen Schnittqualität auch nach längerem Gebrauch sicher.

Ein vorzugsweise zur minimalinvasiven Chirurgie geeignetes z.B. durch einen Trokar in einen Körper einführbares Instrument 10 weist zum Abklemmen von Gefäßen zwei Branchen 19, 20 und ein mittig geführtes Messer 21 auf. Durch die mittige Führung des Messers in zumindest einer der Branchen 19, 20 werden Nachteile vermieden, wie sie sonst infolge außermittiger Trennung des koagulierten Gewebes entstehen könnten. Außerdem ergibt sich durch die saubere mittige Führung des Messers 21 ein verminderter Verschleiß desselben. Die Erfindung ermöglicht somit eine bessere Schnittqualität und eine längere Standzeit des Messers 21.

Eine der Branchen weist einen vorzugsweise keramischen Grundkörper 23 auf, an dessen oberen Rändern nach innen ragende Vorsprünge 38, 39 ausgebildet sind, die die Form eines Hinterschnittes aufweisen. Diese elektrisch isolierenden Vorsprünge erstrecken sich in Richtung zu der Mittelebene M hin und erbringen zwei Verbesserungen: Zum einen wird die Montage der Elektrode 33 erheblich erleichtert. Zum anderen werden zwei Gewebespannstellen geschaffen. Die Vorsprünge können so ausgebildet sein, dass bei geschlossenen Branchen 20, 19 der Abstand A zwischen der durch die obere Branche 20 gebildeten Elektrode und der Elektrode 33 an der Stelle, an der der Abstand A seine größte Weite aufweist, und die beiden Abstände B zwischen den Vorsprüngen 38, 39 und der Branche 20 gleich, nahezu gleich oder geringfügig kleiner als der Abstand A ist. Dies hat den Vorteil, dass während des Koagulationsprozesses, bei dem das Gewebevolumen im Koagulationsbereich abnimmt, das Gewebe trotzdem sicher gehalten wird. Der Abstand A verjüngt sich vorzugsweise ausgehend von den Vorsprüngen 38, 39 bis zu dem Führungsschlitz 31 hin. Bei dem vorliegenden Instrument wird die Koagulationsbreite durch die isolierenden Vorsprünge 38, 39 bestimmt und ist somit sicher reproduzierbar dies verbessert die Koagulationsqualität erheblich.

Ein erfindungsgemäßes Instrument 10 zur Gefäßfusion und Trennung weist eine erste Branche 19 mit einer ersten Elektrode 33 und eine zweite Brache 20 auf, die auf die erste Elektrode 33 zu und von dieser weg beweglich gelagert ist, wobei die zweite Branche 20 einen Längsschlitz 31 aufweist. In diesem ist ein Messer 21 bezüglich des Schafts 11 längs beweglich angeordnet. Dem Messer 21 ist ein Vertikalstellmechanismus 54 zugeordnet, um das Messer 21 in Abhängigkeit von seiner Längsbewegung senkrecht zu der Längsbewegung zu verstellen. Dadurch wird ein vorzeitiges Abstumpfen der Spitze 28b oder der Gleitkante 28a des Messers 21 durch Berührung des keramischen Grundkörpers oder von Kanten oder der Stufe 55 vermieden.

### Bezugszeichenliste:

- 10: Instrument
- 11: Schaft
- 12: Gehäuse
- 13: Handgriff
- 14: Betätigungselemente
- 15: Griffteil
- 16: Betätigungsknopf oder -Hebel
- 17: Kraftübertragungsmittel
- 18: Anwendungsteil, Kopf
- 19: erste Branche
- 20: zweite Branche
- 21: Messer
- 22: Pfeil
- 23: Grundkörper
- 24: Ansatz
- 25: Führungsschlitz
- L: Längsrichtung und Vorschubrichtung
- 26, 27: Schlitzflanken
- S: Spiel zwischen 25 und 21
- D: Dicke des Messers 21
- DK: Dicke der Klinge 28
- 28: Klinge
- 28a: Gleitkante
- 28b: Spitze
- 28c: Schneidkante
- 28d: spitzenfernes Ende der Gleitkante 28a
- 29: Führungsleiste
- 30: Scharnierbereich
- 31: Längsschlitz
- S1: Spiel zwischen 31 und 21
- 32: Ausnehmung
- 33: Elektrode
- 34: endständiger Bereich des Grundkörpers 23
- 35, 36: obere Ränder
- 37: Haltemittel
- 38, 39: Rastvorsprünge
- 40: Elektrodenfläche
- 41: Vorsprung
- A: Weite des Abstandsspalts zwischen 33 und 40
- B: Abstand zwischen 38, 39 und 40
- 42, 43: Flanke der Branche 20
- 44: Leiter
- 45: Zuleitung
- 46: Isolierung
- 47: seitliche Öffnung des Grundkörpers 23
- 48: vorderes Ende von 33
- M: Mittelebene
- 49: Längsausnehmung
- 52, 53: Schlitzflanken von 31
- 54: Vertikalstellmechanismus
- 55: Stufe
- 56: Kurvenfläche
- 57: Kurvenfolgerfläche
- H1: Höhe der Kurvenfläche 56 und somit Verstellweg
- H2: Höhe der Kurvenfolgerfläche 57
- H3: Höhe der Stufe 55
- H4: Abstand zwischen der Gleitkante 28a und der Fläche 58
- 58: Fläche

## Patentansprüche

1. Instrument (10) zur Gefäßfusion und Trennung,
mit einem länglichen Schaft (11), der sich von einem Gehäuse (12) weg erstreckt,
mit einer ersten Branche (19), die eine erste Elektrode (33) aufweist,
mit einer zweiten Branche (20), die auf die erste Elektrode (33) zu und von dieser weg beweglich gelagert ist, wobei die zweite Branche (20) einen Längsschlitz (31) aufweist,
mit einem Messer (21), das bezüglich des Schafts (11) längs beweglich angeordnet und in den Längsschlitz (31) hineinragend oder in diesen einfahrbar angeordnet ist, wobei dem Messer (21) ein Vertikalstellmechanismus (54) zugeordnet ist, um das Messer (21) in Abhängigkeit von seiner Längsbewegung senkrecht zu der Längsbewegung zu verstellen, wobei zu dem Vertikalstellmechanismus (54) eine Kurvenfläche (56) und eine bezüglich des Messers (21) ortsfest angeordnete Kurvenfolgerfläche (57) gehören,
wobei die Kurvenfläche (56) und die Kurvenfolgerfläche (57) schräg zu der Vorschubrichtung (L) orientierte Flächen sind,
und der Verstellmechanismus (54) einen Verstellweg (H1) festlegt, der größer ist als die Höhe (H3) einer an einem Ende der Elektrode (33) ausgebildeten Stufe (55), **dadurch gekennzeichnet, dass** die Kurvenfläche (56) bezüglich des Schafts (11) ortsfest angeordnet ist.

2. Instrument nach einem der vorstehenden Ansprüche, **dadurch gekennzeichnet, dass** dass die erste Branche (19) einen elektrisch isolierenden Grundkörper (23) mit einer rinnenartigen Ausnehmung (32) und einen Führungsschlitz (25) aufweist, wobei an dem Grundkörper (23) Haltemittel (37, 38, 39) zur ortsfesten Lagerung der ersten Elektrode (33) angeordnet sind, die gekrümmt ausgebildet ist,

3. Instrument nach einem der vorstehenden Ansprüche, **dadurch gekennzeichnet, dass** der Vertikalstellmechanismus (54) dazu eingerichtet ist, das Messer (21) bei einer entlang der ersten Elektrode (33) gerichteten Vorschubbewegung senkrecht zu der Vorschubbewegung auf die erste Elektrode (33) hin und bei einer entlang der ersten Elektrode gerichteten Rückzugsbewegung senkrecht zu der Rückzugsbewegung von der ersten Elektrode (33) weg zu bewegen.

4. Instrument nach einem der vorstehenden Ansprüche, **dadurch gekennzeichnet, dass** der Vertikalstellmechanismus (54) beim Vorbeigang eines spitzenfernen Endes (28d) der Gleitkante (28a) an einer der ersten Elektrode (33) benachbarten Fuge oder Stufe (55) aktiv ist.

5. Instrument nach einem der vorstehenden Ansprüche, **dadurch gekennzeichnet, dass** das Messer (21) eine gerade, der ersten Elektrode (33) zugewandte und an dieser anliegende, längsverlaufende Gleitkante (28a) aufweist, die sich von einer Gleitkantenspitze (28b) bis zu einem Gleitkantenende (28d) erstreckt.

6. Instrument nach Anspruch 2, **dadurch gekennzeichnet, dass** zwischen der Elektrode (33) und dem Grundkörper (23) eine Fuge oder Stufe (55) ausgebildet ist.

7. Instrument nach Anspruch 5 bis 6, **dadurch gekennzeichnet, dass** der Abstand der Kurvenfolgerfläche (57) von dem Gleitkantenende (28d) wenigstens so groß ist, wie der Abstand der Kurvenfläche (56) von der Fuge oder Stufe (55).

8. Instrument nach einem der vorstehenden Ansprüche, **dadurch gekennzeichnet, dass** das Messer (21) eine stirnseitige Schneidkante (28c) aufweist, die in einem Winkel zu der Bewegungsrichtung des Messers (21) orientiert ist.

9. Instrument nach einem der vorstehenden Ansprüche, **dadurch gekennzeichnet, dass** die zweite Branche (20) durch einen massiven Metallkörper gebildet ist.

10. Instrument nach einem der vorstehenden Ansprüche, **dadurch gekennzeichnet, dass** die zweite Branche (20) an ihrer der Elektrode (33) der ersten Branche (19) zugewandten Seite eine der Form der Elektrode (33) folgende Form aufweist.

11. Instrument nach einem der vorstehenden Ansprüche, **dadurch gekennzeichnet, dass** bei geschlossenen Branchen (19, 20) des Instruments (10) zwischen der Elektrode (33) und der zweiten Branche (20) ein Abstandsspalt mit einer Weite (A) festgelegt ist.

12. Instrument nach einem der vorstehenden Ansprüche, **dadurch gekennzeichnet, dass** die Haltemittel (37) an beiden einander gegenüberliegenden Rändern (35, 36) der Ausnehmung (32) ausgebildet sind.

## Claims

1. Instrument (10) for vessel fusion and separation,
with an elongated shaft (11), which extends away from a housing (12),
with a first branch (19), which has a first electrode (33),
with a second branch (20), which is mounted on the first electrode (33) to be movable towards and away from this,
wherein the second branch (20) has a longitudinal slot (31),
with a blade (21), which is arranged to be movable longitudinally in relation to the shaft (11) and projects into the longitudinal slot (31) or is arranged to be insertable into this,
wherein the blade (21) has an associated vertical adjustment mechanism (54) to adjust the blade (21) in dependence on its longitudinal movement perpendicularly to the longitudinal movement,
wherein the vertical adjustment mechanism (54) includes a cam face (56) and a cam follower face (57) fixedly arranged in relation to the blade (21),
wherein the cam face (56) and the cam follower face (57) are surfaces oriented obliquely to the feed direction (L),
and the adjustment mechanism (54) fixes an adjustment path (H1), which is larger than the height (H3) of a step (55) configured at an end of the electrode (33), **characterised in that** the cam face (56) is fixedly arranged in relation to the shaft (11).

2. Instrument according to one of the preceding claims, **characterised in that** the first branch (19) has an electrically insulating base body (23) with a groove-like recess (32) and a guide slot (25), wherein holding elements (37, 38, 39) are arranged on the base body (23) for fixed mounting of the first electrode (33), which is curved in configuration.

3. Instrument according to one of the preceding claims, **characterised in that** the vertical adjustment mechanism (54) is arranged to move the blade (21) towards the first electrode (33) perpendicularly to the feed movement in the case of a feed movement directed along the first electrode (33) and away from the first electrode (33) perpendicularly to the return movement in the case of a return movement directed along the first electrode.

4. Instrument according to one of the preceding claims, **characterised in that** the vertical adjustment mechanism (54) is active when an end (28d) of the sliding edge (28a) remote from the tip goes past a join or step (55) adjacent to the first electrode (33).

5. Instrument according to one of the preceding claims, **characterised in that** the blade (21) has a longitudinally extending, straight sliding edge (28a) facing the first electrode (33) and abutting against this, which extends from a sliding edge tip (28b) to a sliding edge end (28d).

6. Instrument according to claim 2, **characterised in that** a join or step (55) is configured between the electrode (33) and the base body (23).

7. Instrument according to claim 5 to 6, **characterised in that** the distance of the cam follower face (57) from the sliding edge end (28d) is at least as large as the distance of the cam face (56) from the join or step (55).

8. Instrument according to one of the preceding claims, **characterised in that** the blade (21) has a cutting edge (28c) on the front side, which is oriented at an angle to the direction of movement of the blade (21).

9. Instrument according to one of the preceding claims, **characterised in that** the second branch (20) is formed by a solid metal body.

10. Instrument according to one of the preceding claims, **characterised in that** on its side facing the electrode (33) of the first branch (19) the second branch (20) has a shape following the shape of the electrode (33).

11. Instrument according to one of the preceding claims, **characterised in that** when the branches (19, 20) of the instrument (10) are closed, a spacing gap with a width (A) is fixed between the electrode (33) and the second branch (20).

12. Instrument according to one of the preceding claims, **characterised in that** the holding elements (37) are configured on both opposite edges (35, 36) of the recess (32).

## Revendications

1. Instrument (10) destiné à la fusion et à la séparation de tissus vasculaires, comportant un fût oblong (11) qui s'étend à l'écart d'un logement (12),
comportant une première branche (19) qui présente une première électrode (33), comportant une deuxième branche (20) qui est montée sur la première électrode (33) de façon mobile vers celle-ci et à l'écart de celle-ci, la deuxième branche (20) présentant une fente longitudinale (31),
comportant un couteau (21) qui est disposé de façon mobile longitudinalement par rapport au fût (11) et qui est disposé de manière à faire saillie dans la fente longitudinale (31) ou à pouvoir être introduit dans celle-ci, un mécanisme de réglage vertical (54) étant affecté au couteau afin de régler le couteau (21) en fonction de son déplacement longitudinal perpendiculairement au déplacement longitudinal,
une surface de came (56) et une surface de suiveur de coupe (57) disposée de façon fixe par rapport au couteau (21), appartenant au mécanisme de réglage vertical (54),
la surface de came (56) et la surface de suivi de came (57) étant des surfaces orientées obliquement par rapport à la direction d'avance (L),
et le mécanisme de réglage (54) détermine un chemin de réglage (H1) qui est supérieur à la hauteur (H3) d'un gradin (55) réalisé à une extrémité de l'électrode (33), **caractérisé en ce que** la surface de came (56) est disposée de façon fixe par rapport au fût (11)

2. Instrument selon l'une des revendications qui précèdent, **caractérisé en ce que** la première branche (19) présente un corps de base (23) isolant électriquement comportant une cavité (32) du genre rigole et une fente de guidage (25), des moyens de maintien (37, 38, 39) étant disposés en vue du montage fixe de la première électrode (33), laquelle a une conformation courbe.

3. Instrument selon l'une des revendications qui précèdent, **caractérisé en ce que** le mécanisme de réglage vertical (54) est agencé de façon à déplacer le couteau (21), dans le cas d'un mouvement d'avance orienté le long de la première électrode (33) en direction de la première électrode (33), perpendiculairement au mouvement d'avance et dans le cas d'un mouvement de retrait orienté le long de la première électrode, perpendiculairement au mouvement de retrait à partir de la première électrode (33).

4. Instrument selon une des revendications qui précèdent, **caractérisé en ce que** le mécanisme de réglage vertical (54) est actif lors du passage d'une extrémité (28d) située loin de la pointe, l'arête de glissement (28a) est active sur une jointure ou un gradin (55) voisins de la première électrode (33).

5. Instrument selon une des revendications qui précèdent, **caractérisé en ce que** le couteau (21) présente une arête de glissement (28a) longitudinale, orientée vers la première électrode (33) et en appui contre celle-ci, qui s'étend d'une pointe d'arête de glissement (28b) jusqu'à une extrémité d'arête de glissement (28d).

6. Instrument selon la revendication 2, **caractérisé en ce qu'**entre l'électrode (33) et le corps de base (23) une jointure ou un gradin (55) se trouve formé.

7. Instrument selon la revendication 5 à 6, **caractérisé en ce que** l'écart de la surface de suivi de came (57) par rapport à l'extrémité (28d) d'arête de glissement est au moins aussi important que l'écart de la surface de came (56) par rapport à la jointure ou au gradin (55).

8. Instrument selon l'une des revendications qui précèdent, **caractérisé en ce que** le couteau (21) présente une arête de coupe (28c) frontale qui est orientée avec un certain angle par rapport à la direction de déplacement du couteau (21).

9. Instrument selon l'une des revendications qui précèdent, **caractérisé en ce que** la deuxième branche (20) est constituée d'un corps métallique massif.

10. Instrument selon l'une des revendications qui précèdent, **caractérisé en ce que** la deuxième branche (20) présente sur son côté orienté vers l'électrode (33) de la première branche (19) une forme qui suit la forme de l'électrode (33).

11. Instrument selon une des revendications qui précèdent, **caractérisé en ce que** les branches (19, 20) de l'instrument (10) étant fermées, une fente d'écart d'une largeur (A) est déterminée entre l'électrode (33) et la deuxième branche (20).

12. Instrument selon l'une des revendications qui précèdent, **caractérisé en ce que** les moyens de maintien (37) sont formés sur les deux bord (35, 36) qui se font face, de la cavité (32).
